Europäisches Patentamt

European Patent Office

Office européen des brevets

(19)

(11) Publication number: **0 124 285**
**A2**

# (12) EUROPEAN PATENT APPLICATION

(21) Application number: 84302212.0

(22) Date of filing: 30.03.84

(51) Int. Cl.³: **C 12 Q 1/16**

(30) Priority: 31.03.83 GB 8309059
23.12.83 GB 8334326

(43) Date of publication of application: 07.11.84
**Bulletin 84/45**

(84) Designated Contracting States: **AT BE CH DE FR GB IT LI LU NL SE**

(71) Applicant: **Silman, Robert Edward, 83 Highgate West Hill, London N6 (GB)**
Applicant: **Tabaqchali, Soad, 33 Dukes Avenue, Chiswick London W4 (GB)**
Applicant: **Holland, Diane Thelma, 20 Franklin Street, Greymouth South Island (NZ)**

(72) Inventor: **Silman, Robert Edward, 83 Highgate West Hill, London N6 (GB)**
Inventor: **Tabaqchali, Soad, 33 Dukes Avenue, Chiswick London W4 (GB)**
Inventor: **Holland, Diane Thelma, 20 Franklin Street, Greymouth South Island (NZ)**

(74) Representative: **Colgan, Stephen James et al, CARPMAELS & RANSFORD 43 Bloomsbury Square, London WC1A 2RA. (GB)**

(54) **Method and device for detecting microorganisms.**

(57) The presence of microorganisms, such as bacteria, in a sample is determined by contacting the sample with an emissive agent, for example a radioactive amino acid; separating off the excess emissive agent after an appropriate incubation period; and detecting the level of emission from the residue, for example with a radiation counter. The emissive agent may be carried in an absorbent member of a device such as a dipstick or reagent strip.

## METHOD AND DEVICE FOR
## DETECTING MICROORGANISMS

### Field of the Invention

The present invention relates to a method and a device for determining the presence of microorganisms in a sample, for example either a sample that is, or is derived from, a specimen of fluid from a human or animal body or a sample that is derived from water or foodstuff that may have been contaminated.

### Background to the Invention

It is known that in order to diagnose an infection in a human or animal body, or to determine whether there is microbial contamination of water, food or the like, a specimen is obtained which is then subjected to examination. The conventional techniques for effecting such an examination are complex and time-consuming, as may be illustrated by the following brief description of the conventional examination of a sample of cerebrospinal fluid (CSF).

Firstly, the CSF is subjected to various investigations that may provide indirect evidence of the presence of an infection in the patient from whom the sample was taken. Such investigations include a cell count, in particular for the white cell and the red cell content (which is carried out by microscopy) as well as a protein estimation and a sugar estimation. Preparations may also be carried out with a view to determining the presence of yeasts, e.g. Cryptococci. However, a negative result in these investigations does not imply the absence of infection and further examination is then necessary, as follows.

The CSF is centrifuged and the resultant deposit is divided into portions. One portion may be spotted on a microscope slide, dried and subjected to Gram staining, following which examination may reveal evidence of pus cells and/or of infecting microorganisms (specifically bacteria). Another portion of the centrifuge deposit may be subjected to Ziehl-Neelsen staining, which is intended to reveal the presence of acid-fast microorganisms.

However, negative results in the aforesaid staining tests cannot be regarded as conclusive evidence of the absence of an infection and it is necessary to culture the centrifuge deposit on a variety of different media and under various (e.g. aerobic, carbon dioxide or anaerobic) conditions, as well as on media for mycobacteria, since any one given medium and any one set of conditions will not support the growth of microorganisms of all kinds. Bacterial colonies that are formed during the culture period are identified, by means of staining, various biochemical techniques and the determination of antibiotic sensitivities, the identification taking typically another 24 hours or more.

There is clearly a need for a method of determining the presence of microorganisms in a sample that is as reliable as the conventional methods and yet which can be performed more rapidly.

In medical and veterinary practice, it is often necessary to determine whether or not a given compound is present in a specimen. Many tests in clinical chemistry have now been reduced to the use of a reagent strip or "dipstick", i.e. a stick or strip, usually of a plastics material, to which a pad is attached that has been impregnated with one or more active ingredients.

For example, one can test for the presence of sugar in a sample of urine by immersing the pad of an appropriate dipstick in the sample for a few seconds. After the dipstick has been removed from the sample, the pad will change colour if the active ingredient or mixture of active ingredients has reacted with sugar in the sample. The content of sugar in the sample may be estimated by comparing the colour of the pad with the colours on a chart that specifies a sugar value or range of sugar values for each colour. The colour chart is usually included in the package in which the dipsticks are marketed.

Dipstick systems are available for the testing of a wide variety of compounds, for example proteins, ketones, bilirubin and haemoglobin, each dipstick system having a particular active ingredient or combination of active ingredients and its own colour conversion chart. Of course, it is also possible to arrange a series of pads on a single stick to provide a multi-purpose dipstick that one can use to test for several substances simultaneously.

Summary of the Invention

The present invention provides a method of determining the presence of microorganisms in a sample, which method comprises the steps of bringing an emissive agent into contact with at least part of the sample; separating off any excess emissive agent; and detecting the level of emission from at least part of the residue.

The detected level of emission serves as an indicator of the presence or absence of microorganisms. In many cases, only a coarse, qualitative determination will be needed; in other words, it may be necessary only to know whether microorganisms were present in or substantially absent from the original sample. In other cases, the method of the present invention may be used to determine quantitatively the level or concentration of microorganisms in the sample. In yet other cases, the present method may be used to determine the presence of microorganisms and their antibiotic susceptibility.

The present method can be readily adapted to a dipstick format, that is to say the active ingredient(s) can be carried on a dipstick (which term herein includes a reagent strip) and the reaction can be confined to the dipstick. Accordingly, the present invention also provides a device for determining the presence of microorganisms in a sample, characterised in that it comprises an emissive agent and a solid carrier for that emissive agent. The presence of microorganisms in a liquid sample may be detected by moistening the solid carrier of a device according to this invention with at least part of the sample, removing excess emissive agent, e.g. by washing the solid carrier, and thereafter detecting the level of emission from the solid carrier.

Brief Description of the Drawings

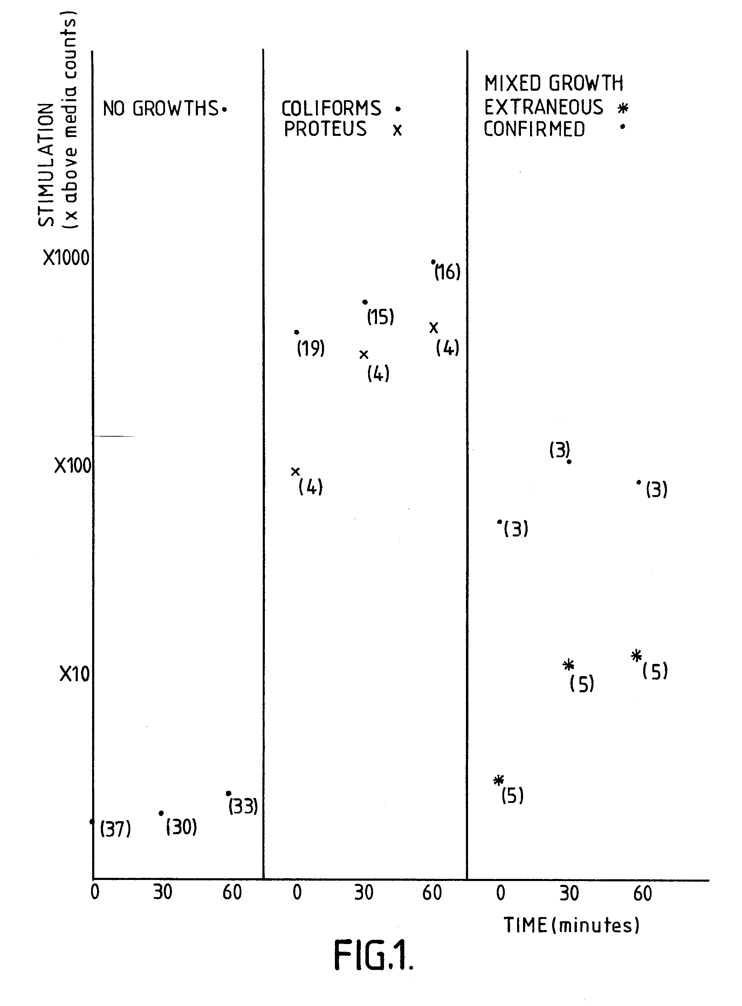

Figures 1, 2 and 3 are graphical representations of results obtained in experiments that are described in the specific Examples hereinafter.

Description of Preferred Embodiments

A material to be tested that is not in liquid form is usually made up as a (preferably aqueous) solution or dispersion to provide the sample.

Although the sample may be taken directly from a specimen of fluid from a human or animal body, for example urine, cerebrospinal fluid, serum, whole blood, lymph or other aspirate, it may be preferred first to centrifuge the fluid, discard the resultant supernatant liquid and take the sample from the remaining matter. Of course, the fluid may be subjected to prior treatment as appropriate; for example in the case of whole blood it is usually desirable to separate off the cells from any microorganisms that may be present, for example by differential centrifugation or differential filtration.

The use of an emission, such as radioactivity, fluorescence or the like, is necessary in the present invention in order to be able to detect products, such as proteins, that are present in minute quantities, possibly of the order of picograms or femtograms. It is preferred that the emissive agent should contain a radioactive element, for example $^{35}S$, $^{32}P$, $^{14}C$, $^{3}H$ or $^{125}I$. In principle, the emissive agent may be any compound that can be utilized in the metabolic processes of a microorganism, e.g. a sugar (such as glucose, fructose, maltose or lactose), an organic acid (such as acetic acid or succinic acid), a hydrocarbon, an amino acid or other compound that can be incorporated into protein, a compound that can be converted by metabolism into an amino acid, or even an inorganic salt (such as a phosphate or sulfate). The use of a radioactive amino acid as the emissive agent is especially preferred, examples being $^{3}H$ leucine, $^{14}C$ lysine, $^{14}C$ leucine and $^{35}S$ methionine.

It will be convenient first to describe embodiments of this invention that do not entail the use of a dipstick and in which the sample and the emissive agent are brought into contact by forming a mixture thereof. The emissive agent is conveniently in liquid form, e.g. a solution or dispersion in, preferably, an aqueous medium.

If appropriate, a nutrient medium may be incorporated in the mixture of the sample and the emissive agent. The nutrient medium may be selected from any of the customary media, for instance nutrient broth, brain-heart infusion or Eagle's medium, although the precise selection will generally be influenced by an assumption of the type of microorganisms that might be present. If such is used, the order in which the sample, the nutrient medium and the emissive agent are mixed together is believed not to be critical. In many cases, however, it has been found that the use of a nutrient medium may be dispensed with, this being clearly desirable from the viewpoint of economy both of time and materials. For instance, the method has been shown to work with urine samples without the addition of any nutrient medium.

The mixture of sample and emissive agent may then be incubated. The incubation may be effected conveniently at room temperature (15-20°C), although it is also possible for it to be effected at a moderately elevated temperature, for example up to 37°C. The period of incubation can be comparatively short: good results have been obtained with incubation periods of one or two hours down to, for example, thirty, fifteen or even five minutes or less. Indeed, in many cases satisfactory results have been obtained by separating off the excess emissive agent directly after forming the mixture of sample and emissive agent, the incubation period in such cases being negligible.

When microorganisms are present in a sample treated according to the present invention, it appears that at least some of the emissive agent is somehow "fixed" by the microorganisms. As the precise mechanism has not been determined, it is intended that the term "fixed" should be construed broadly, to include incorporation in (for example by translation or other metabolic processes) or attachment to the microorganisms and/or their products, especially their protein products, thereby labelling them. Before a determination is made, the excess (or "unfixed") emissive agent is separated off from the emissively labelled substances. Where the emissively-labelled substances are solids and the excess emissive agent remains in solution, separation may be readily effected, for example by centrifugation followed by removal of the supernatant liquid (as by decanting or by pipetting), or by filtration. Other separation techniques that may be employed include dialysis, adsorption or binding.

In a number of cases, however, it may be necessary to precipitate emissively labelled substances. For example, it may be appropriate to precipitate proteins in the mixture by addition of a suitable precipitant, for example trichloroacetic acid (TCA), an alcohol (e.g. methanol or ethanol) or ammonium sulphate.

Clearly, the better the separation of the excess emissive agent from the fixed emissive agent, the more accurate will be the determination. However, in practice, and especially when only a qualitative determination is required, it will not usually be necessary to take special steps to ensure removal of the last traces of excess emissive agent. Similarly, a minor loss of emissively labelled product during the separation stage will not normally vitiate the determination and can therefore be tolerated, unless a highly accurate, qualitative determination is required.

If a nutrient medium is employed, it may be desirable that it should be free of the non-emissive analogue of the emissive agent used: for instance, if the emissive agent is $^{35}$S-methionine, the nutrient medium could be methionine free. Thus, the emissive agent will not be "competing" against the unlabelled analogue and this ensures that an adequate proportion of the emissive agent is fixed.

After the excess emissive agent has been separated off, the residue is placed in a suitable apparatus and the emission therefrom is sensed by means adapted to provide a reading of the emission level. Thus, for example, when the emissive agent is radioactive, the emission may be detected by a suitable radiation counter.

When the emissive agent produces beta-radiation, it may be desirable to add a scintillant and to count the emission using a scintillation counter. Suitable scintillants are well known and commercially available, and include 2,5-diphenyloxazole (PPO), 1,4-bis-2-(5-phenyloxazoyl)-benzene (POPOP), 1,4-bis-2-(4-methyl-5-phenyloxazoyl)-benzene (Dimethyl-POPOP) and 2-(4-tert-butylphenyl)-5-(4-biphenylyl)-1,3,4-oxadiazole (Butyl-PBD). The scintillants may be used in the form of a toluene solution, which may include, for instance, a surfactant, such as Triton (trade name), especially when the scintillant is to be used in an aqueous system. However, it is also possible and indeed may be preferred, to dispense with the use of a scintillant and to measure the radiation directly, for example with a Geiger counter or the like.

Another preferred technique is to determine the level of radioactive emission from the filtration residue using a detecting device such as is described in European Patent Application No. 83307104.6 in the name of B.R. Pullan, the disclosure of which application is incorporated herein by reference. One advantage of the Pullan apparatus is that it permits the emission from a number of samples to be detected simultaneously, without the need to add a scintillant.

In general, a high reading of emission intensity is indicative of an infection in the patient from whom the sample was taken (or of microbial contamination where the sample is from a water supply, a foodstuff or the like), whereas a low reading is indicative of the absence of infection (or microbial contamination).

An interesting modification of the procedure described above is to determine the antibiotic susceptibility of the microorganism in the sample. Thus, a number of samples, e.g. of urine, from the same patient may be prepared (conveniently by dividing a single specimen into a number of portions), each sample then being incubated in the presence of a respective one of a series of different antibiotic agents and, if required, in the presence of a suitable nutrient medium. An emissive agent is then added to each sample mixture and the degree to which the activity of the microorganism has been arrested by the antibiotic agent is then determined by separating off the excess emissive agent and detecting the emission from the residue, in the manner described above. Thus, if infection is present in the patient, then this procedure will provide an indication of the drug sensitivity of the organism responsible for that infection. As this procedure involves the detection of emission level from a plurality of samples, a multiple scanning or detecting apparatus, such as the Pullan apparatus referred to above, will be particularly useful.

A further modification of the above-described method may be used to estimate the level of antibiotic that is being achieved in a patient undergoing treatment and to determine whether that level attained in the serum is adequate to inhibit the growth of the infecting organism, e.g. back-titration in cases of endocarditis. A specimen of fluid, e.g. serum, CSF or urine, is taken from the patient. Multiple dilutions of the specimen are made and a standard inoculum of the infecting organism is added to each dilution. A sample of each resultant mixture is then mixed with the emissive agent and (if appropriate) a nutrient, incubated (if appropriate) and subjected to the separation step, the emission level from the residue then being detected. The result may then be compared with that obtained in a control experiment, using control standard organisms and a control from which the body fluid was omitted. The inhibition on the microorganism growth will determine the dilution at which the infecting organism is inhibited and monitor therapy. This in turn gives an indication of the level of antibiotic in the original specimen taken from the patient.

The present method may also be used in the in vitro determination of minimum inhibitory concentrations (MIC's). Different dilutions of an antibiotic are added to a series of samples, each containing a standard inoculum of the microorganism to be tested, an emissive agent, and optionally, a nutrient medium. The inhibition of microorganism activity in a sample is then ascertained by the steps of incubation (if appropriate), separation and the determination of emission level, as described above.

It has also been found that for a given volume of specimen, a given quantity of $^{35}$S methionine (or other emissive agent) and a given incubation time, the level of radioactive emission of the retentate may give an indication of the type of microorganism infecting the specimen. For instance, E. coli gave higher counts in a number of experiments than did Streptococcus. In other words, the rate of incorporation seemed to be related to the type of infection as much as to the degree of infection.

The above embodiments of the present method can be readily adapted to the use of an emissive agent on or within a solid carrier, in particular a device according to the present invention wherein the carrier is an absorbent member and is impregnated with the emissive agent.

The absorbent member is conveniently a non-woven fibrous material, preferably paper. The preferred fibres are glass fibres, e.g. microfibres, although it would be possible to use other fibres, such as those of natural or regenerated cellulose. An especially preferred absorbent material is a Millipore filter material.

The impregnated absorbent member can be used alone. Thus, for example, it would be possible to form the device according to the invention simply as a strip of absorbent material, e.g. paper, impregnated with the emissive agent. However, this may be wasteful of the emissive agent and it is therefore preferred to employ an absorbent member that is associated with a support. This permits the economical use of the emissive agent by, for instance, forming the absorbent member as a small pad attached to a strip or stick of a suitably inert material such as wood or a plastics material; the pad may be secured to the support in any appropriate manner, for example by means of an adhesive and/or by means of an overlying permeable material that holds the pad against the support.

The dimensions of the device are not critical: typical supports may be about 5-10 mm wide, 0.2-1 mm thick and 7-12 cm long, the pads having a width equal to the width of the support and a length of 1 to 3 times that width. Alternatively, the absorbent member may be formed as a small wad held within the bore of a small tube e.g. of a clear, transparent material such as glass or

0124285

- 12 -

Perspex (trade mark); here, the frictional engagement of the absorbent member with the inner wall of the tubular support may be sufficient to hold the absorbent member in place.

When manufacturing the device according to the present invention, the absorbent member is preferably impregnated with a dilute solution of the emissive agent and then dried. However, it has been found that the drying operation may decrease the efficiency of the device when $^{35}$S methionine is used as the emissive agent. This decrease in efficiency manifests itself, in the case of infected samples, in counts that are lower than expected and, in the case of non-infected samples, in counts that are higher than expected. It is thought that this is due to the oxidation of the sulphur during drying. Thus, it may prove advantageous either to keep the $^{35}$S methionine-containing member moistened with a reducing agent, or to dry the $^{35}$S methionine-containing member under non-oxidising conditions, for example in a vacuum or in an inert gas such as nitrogen. Alternatively, it is possible to employ a sulphur-free emissive agent labelled with another radioactive element, such as $^{14}$C or $^3$H. $^{14}$C-leucine is a preferred emissive agent: it is particularly preferred to employ $^{14}$C-leucine in the presence of glycerol, the latter being used typically in an amount of 5% by weight.

The device of the present invention can readily be employed with any of a wide variety of samples, especially those mentioned above, in order to bring the emissive agent and sample into contact. For example, the absorbent member may be dipped into a urine sample for a few minutes, removed and then washed, e.g. with distilled water. After the unreacted emissive agent has been washed away, the absorbent member, after drying, is placed in a suitable apparatus and the emission therefrom is sensed by means adapted to provide a reading of the emission level. When the emissive agent is radioactive, the emission may be detected by a suitable radiation counter, such as a Geiger counter, scintillation counter or Pullan counter, as discussed above.

If more than one absorbent member is used per sample, then one can employ the technique to determine the antibiotic sensitivity of the microorganism. One absorbent member would be impregnated with the emissive agent without antibiotic while the other (or others) would be impregnated with the emissive agent plus an antibiotic. The number of concurrent tests performed on each sample would be determined by the variety of antibiotics one wished to check and by their range of concentration. Effectively, the antibiotic sensitivity of the microorganism would be a function of the difference in incorporation when the emissive agent is used without antibiotic compared with when it is used together with an antibiotic. Though several absorbent members may be used for such a test, they can be carried on a single stick (or other support material).

Although it would be possible to impregnate the absorbent member with an antibiotic as well as with the emissive agent, it is considered that this may not be the only method of determining the antibiotic sensitivity of any microorganism whose presence is detected by means of this invention. The living microorganism will, in general, react with the emissive agent before it is completely affected by the bacteriocidal effects of the antibiotic. It may, therefore, be more informative to preincubate the microorganism-containing specimen with the antibiotic prior to any reaction with the emissive agent on the device according to this invention.

An important application of this invention would be to add the absorbent member to the sample as soon as possible after the sample is taken. Indeed, it may be possible to house the absorbent member within the sterile container in which the sample is to be collected. The immediacy of the test would reduce the chance of an infecting microorganism, whose viability is difficult to preserve outside the site of its infection, dying and it would also reduce the chance of an extraneous microorganism,which has been introduced into the sample by contamination from another source, taking hold. In other words, the chance of false negative or of false positive results can be reduced by the immediacy of the test, unlike many other forms of clinical laboratory analysis where the bacteriological sample which is taken from the patient may not be the same as that which arrives for analysis within the laboratory. The technique described herein allows one to take the test to the sample as well as taking the sample to the test. In principle, when the test is complete (i.e. short term incubation of sample with absorbent member impregnated with an emissive agent followed by washing), there is nothing to prevent the radioactive proteins which have attached to the absorbent member from being counted at another location or at another time.

The present invention is especially useful for determining the presence of bacteria in a sample. However, the invention is applicable to other types of microorganism, for example fungi (which term herein includes the yeasts).

Although the present invention has been described herein primarily in the context of determining the presence of microorganisms in a sample of, or derived from, a body fluid, the present method is not confined to the field of diagnostic medicine. Thus, the determination of the level of microorganisms in a sample is a necessary procedure in many industrial fields, for example in the treatment of potable water, in the treatment of sewage and other waste and in the preparation of foodstuffs and beverages. After the presence of microorganisms has been established by means of the present invention, it is then possible to identify those microorganisms either by conventional microbiological methods or by using the techniques described by R.E. Silman in European Patent Specification No. 0,077,149A, the teaching of which is incorporated herein by reference. Thus, for instance, radioactive proteins may be eluted from the absorbent member of a device of this invention in order to generate an identifier for the infecting microorganism that can be processed in the manner taught by R.E. Silman.

The present invention is illustrated in and by the following Examples.

The $^{35}$S methionine used in these Examples was obtained from Amersham International, U.K., as a solution in 20 mM aqueous potassium acetate containing 0.1% of 2-mercaptoethanol, the specific activity ranging from 600 to 1450 mmol with a concentration greater than 10 mCi/ml.

Example 1

A specimen of urine was taken from a human patient and was centrifuged, the resultant supernatant liquid being then discarded. A 25 µl sample was taken from the slurry remaining after centrifugation and was placed in a microvial together with 25 µl of Eagle's medium. One µl of $^{35}$S methionine (approx. 10 µCi) was then added to the mixture and the resultant sample was then left to incubate at room temperature for thirty minutes. After incubation, 1 µl of the sample was taken into 50 µl BSA (bovine serum albumen) (1 mg per ml) and then 1 ml of 10% TCA was added to precipitate the proteins therein. The sample was then filtered, the precipitated proteins being collected on a glass-fibre paper.

The glass-fibre paper, together with the proteins collected thereon, was placed in a scintillation vial and 10 ml of a scintillant (PPO) solution were added. The scintillation vial was then placed in a Packard (trade name) scintillation counter and a reading taken. The reading gave a high count for the emission, thereby indicating the presence of an infection in the patient.

Example 2

A series of experiments were carried out using the following procedure.

A specimen of urine was well mixed and a 50 µl sample was taken, to which sample there were added 50 µl of Eagle's medium containing 1 µl of $^{35}$S-methionine (approx. 10 µCi). An intimate blend was formed by means of vortex mixing. After a prescribed period of time, a 25 µl aliquot was taken from the mixture, which aliquot was boiled for ten minutes to render it sterile. One µl of the boiled mixture was spotted onto a glass-fibre filter paper and washed with 20 ml of a saline solution. Residual saline solution was removed with methanol and the filter paper was then dried at 56°C for ten minutes. The dried paper was placed in a counting vial to which 10 ml of scintillant (PPO/POPOP/toluene) were added. The vial was capped and then placed in a Packard scintillation counter and the emission was counted for one minute and the count was recorded.

Each urine specimen investigated by this method was also passed to laboratory technicians for determination and identification by conventional techniques. The results obtained according to the method of the present invention were then grouped together according to the results of the conventional techniques, namely the group of aliquots in which no significant level of microorganisms was found (the "no growth" group), the group in which significant levels of bacteria were found (more than $10^5$ organisms per ml of urine), i.e. Coliform bacteria and Proteus bacteria, and a group in which a mixture of bacteria were found (the "mixed growth" group).

The results in the mixed-growth group were subdivided; some were classed as extraneous since they were probably due to contamination of the urine specimen in question by bacteria from a source other than the site of infection in the patient. The others were classed as confirmed growth, since they were considered to be causative organisms, i.e. causing an infection.

The results are shown in Figure 1, which Figure shows the average stimulation of the scintillation counter by the samples taken after a prescribed period, the stimulation being plotted against the time (in minutes) elapsing between vortex-mixing and the taking of the 25 μl aliquot for testing. In those experiments wherein the aliquot was taken immediately after vortex mixing, the elapsed time is regarded as zero (0). The number in brackets adjacent a point indicates the number of experiments from which the average value was taken.

It should be noted that the stimulation is expressed, on a logarithmic scale, as a factor of the stimulation obtained in the control (a mixture of Eagle's medium and $^{35}$S-methionine without addition of the urine specimen).

It can be seen from Figure 1 that the results obtained by the present method correlate extremely well with the results obtained by the conventional techniques. Thus, whereas the "no-growth" samples gave average counts of from 0.9 to 1.6 times the control count, the lowest average count obtained for the Proteus bacteria, for example, was about 95 times the control count. Thus, the present invention offers a method of determining the presence or absence of microorganisms that is extremely sensitive and yet which can be carried out simply, quickly and reliably.

Example 3

In order to evaluate the method as a means for determining antibiotic susceptibility, a series of experiments were carried out using the following procedure.

50 µl of urine taken from a patient with a bacterial infection were mixed with 50 µl of Eagle's medium and a prescribed amount of an antibiotic (specifically Ampicillin). The resultant mixture was incubated for one hour at room temperature, following which 1 µl of $^{35}$S-methionine (approx. 10 µCi) was added. The whole was intimately blended by vortex mixing and, after prescribed periods of time, aliquot samples were taken and tested in a manner analogous to that described in Example 2 above.

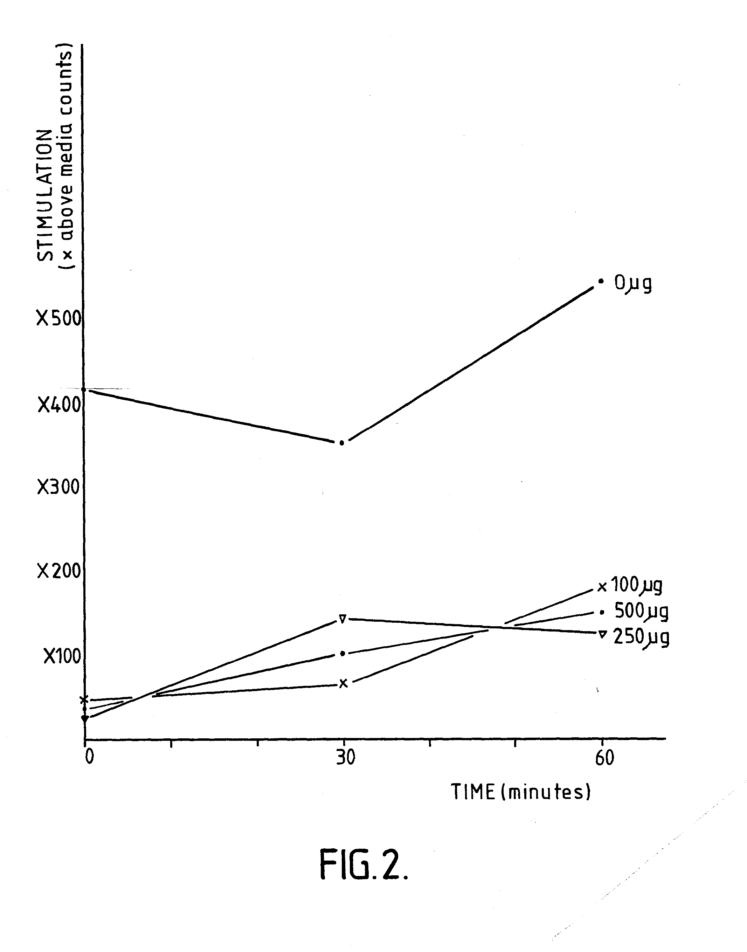

The results are summarised in Figure 2, in which stimulation of the scintillation counter is plotted against the time that elapsed from vortex mixing to the taking of the aliquot for testing. As in Figure 1, the stimulation is in counts expressed as a factor of the counts obtained in the control.

Figure 2 shows the results obtained in three series of experiments using, respectively, 500 µg, 250 µg, 100 µg of Ampicillin in the sample, together with the results of experiments in which no (0 µg) Ampicillin was added. It will be seen that the presence of Ampicillin inhibited the activity of the microorganism in the urine specimen, which microorganism was shown by conventional techniques to have moderate sensitivity to Ampicillin.

Example 4

A number of reagent devices according to the present invention were made in the following manner. A reagent solution was prepared by dissolving 1 µl of $^{35}$S methionine (approx. 10 µCi) in 100 ml of water. A paper disc (2.5 cm diameter) was then immersed in the resultant solution, withdrawn and blotted. Eight $^{35}$S methionine-impregnated discs were made from each of six types of paper, namely Whatman No. 1 filterpaper, Whatman 3 mm chromatography paper, Millipore .22 µ paper, and glass-fibre papers designated as GF/C, GF/D and GF/F.

The various types of paper differed in both thickness and absorbence; however, it was estimated that 100-200 µl of solution was taken up by each disc. Thus, each paper disc was impregnated with approximately 0.001-0.002 µl of the original $^{35}$S methionine.

A set of urine specimens were taken, some of which were infected, and one disc (blotted but still wet) of each type of paper was left in contact with 500 µl of each urine sample for thirty minutes at room temperature.

The discs were then removed and washed with water and then with methanol on a vacuum tower. After drying, each disc was placed in a respective scintillation vial, a proprietary scintillant was added and the radiation was counted in a scintillation counter.

The results (in counts per minute) of these experiments are summarised in the following Table. The identification of the culture growth was effected by conventional techniques.

## Table 1

### Emission level (cpm) for each paper disc

| Culture growth | GF/C | Whatman 3 mm chromatography paper | Whatman No. 1 filter paper | GF/D | GF/F | Millipore .22μ |
|---|---|---|---|---|---|---|
| Coliform | 5430 | 2583 | 625 | 30,657 | 8954 | 1270 |
| Mixed | 2953 | 1700 | 590 | 2,476 | 6642 | 2291 |
| Enterococcus | 61 | 191 | 47 | 315 | 135 | 130 |
| Coliform | 65 | 229 | 57 | 1,217 | 177 | 263 |
| Very mixed | 2386 | 926 | 295 | 10,366 | 6289 | 914 |
| No growth | 42 | 241 | 47 | 126 | 74 | 215 |
| Beta-haem strep. | 41 | 386 | 101 | 283 | 268 | 198 |
| No growth | 51 | 205 | 85 | 117 | 59 | 134 |

The degree of infection in the infected urine specimens was not determined by conventional techniques, since the purpose of the experiments was simply to compare the emission levels obtainable with various kinds of paper. All of the papers tested worked to some degree, although the results show that the glass-fibre papers were far more efficient than the other papers tested.

Example 5

A number of reagent devices according to this invention were prepared by dunking pads of glass-fibre paper (GF/C) in a solution of $1\mu l$ $^{35}S$ methionine (approximately 10 $\mu Ci$) in 50 ml of water. Each glass-fibre pad absorbed approximately 200 $\mu l$ of solution, which meant that each pad was impregnated with approximately 0.004 $\mu l$ of $^{35}S$ methionine.

Every specimen of urine that came into a hospital bacteriology department during the course of a normal working day was tested as follows. An impregnated glass-fibre pad, prepared as described above, was placed in a Petri dish and 500 $\mu l$ of urine from a given specimen were then added. After thirty minutes had elapsed, the pad was removed from the urine and washed with water and then with methanol on a vacuum tower. Each experiment was done in duplicate.

After drying, each pad was placed in a scintillation vial to which a scintillant was added. The vial was capped and then placed in a scintillation counter and the emission was counted for one minute and the count was recorded.

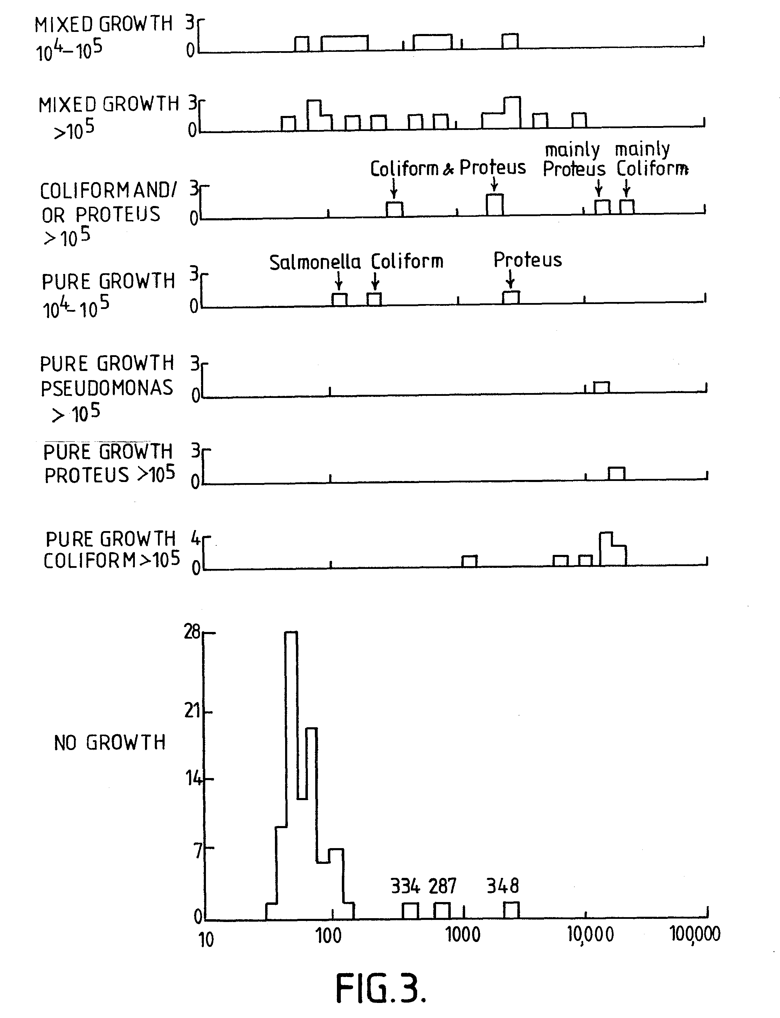

Each urine specimen investigated by means of the above method was also passed to laboratory technicians for determination and identification by conventional techniques. The results obtained according to the method of the present invention are recorded in the accompanying Figure **3**, the results being grouped together according to the results of the conventional investigations in the bacteriology laboratory.

The results are expressed as counts per minute (cpm) along the horizontal axis using a logarithmic scale. The number of samples within any given block of counts are represented along the vertical axis on an arithmetic scale. The results grouped along the bottom column relate to those specimens which were identified in the bacteriology laboratory as having "no growth" (i.e. no infection). The results in the next column relate to those specimens having a clearly identified infection with a pure coliform bacillus at $>10^5$ organisms ("org")/ml. The next column relates to a specimen identified as infected with proteus $>10^5$ org/ml. The next column relates to a specimen identified as infected with pseudomonas $>10^5$ org/ml. The next column relates to samples infected with a pure growth of salmonella, coliform or proteus but at a concentration of $10^4 - 10^5$ org/ml. The next column relates to a mixed growth of $>10^5$ org/ml but where the principal microorganism had been identified as a coliform or proteus. The next column also relates to samples with a mixed growth $> 10^5$ org/ml but where there was no further identification of the infecting microorganism. The last column also relates to samples which had an unidentified mixed growth but at a concentration of $10^4 - 10^5$ org/ml.

Apart from three samples (marked with the reference numbers 348, 287 and 334) in the no-growth group, there was no overlap with the infected samples except in those cases where there were low colony counts or where the results were thought to be due to extraneous infecting organisms.

Regarding the three high counts in the no-growth group, the experiments on sample No. 334 could not be repeated as the urine had been discarded and the patient had been discharged from the hospital. The urine sample No. 348 was recultured in the laboratory and tested again. The second test gave a count of 2283 cpm. as against an original count of 2216 cpm. However, the laboratory technicians were this time able to culture a lactose-fermenting organism and also, possibly, some coliforms. Further investigation was not possible as the patient had also been discharged.

The patient from whom sample No. 287 had been collected was found still to be in hospital. She had been receiving ampicillin for a parotid gland infection. A further specimen of urine from this patient was tested in the above-described manner and a reading of 843 cpm (counts per minute) was obtained, as against a reading of 820 cpm with the first sample. This time, however, the investigation in the bacteriology laboratory showed a full-scale urinary infection with coliform bacteria having a colony count greater than $10^5$, the microorganism being resistant to ampicillin and sensitive to septrin. Further experiments were carred out in which the urine was pre-incubated with various concentrations of ampicillin and of septrin for one hour, each sample then being tested using a $^{35}$S methionine-impregnated glass-fibre pad in the manner described above. It was found that at the lowest concentration of septrin (approximately 320 µg) the counts were less than 10% of those found when the same specimen contained no antibiotic. However, at the lowest concentration of ampicillin (approximately 500 µg) the counts were the same as if there had been no antibiotic present.

Ignoring the sample No. 334, the two remaining tests in the original series that appeared to be "false positives" (i.e. the tests of samples No. 348 and No. 287) were shown subsequently to be "true positives". Excluding samples Nos. 287 and 348, the results can be tabulated as shown in Table 2.

Table 2

Statistical analysis of results in Example 5

|  | Mean | Standard deviation |
|---|---|---|
| No growth | 76 | 46 |
| Pure growth $\Rightarrow 10^5$ | 12,560 | 5,527 |
| Mixed growth $> 10^5$ | 4,432 | 6,727 |
| Pure or Mixed growth $10^4$–$10^5$ | 463 | 668 |

No growths are significantly different from Pure growth $> 10^5$ ($p < 0.001$) and from Mixed growth $> 10^5$ ($p < 0.01$).

Discussion

The use of the emissive agent is necessary in order to be able to distinguish the signal from the bacteria from proteins that may be present in the sample from other sources. For example, the urine or other material to be tested may have a significant quantity of protein that is unrelated to any microorganism that may be present. It is possible only for a living organism with an active genetic apparatus to synthesise proteins (or other products) from the emissive agent and, therefore, "noise" from non-living matter is excluded. Since microorganisms are far more active than other forms of living tissue, such as white cells, any signal that is obtained will be due primarily to the microorganisms that were present in the sample.

The present invention does not require the residue comprising the radiolabelled proteins (or other emissive products) to be separated (e.g. by electrophoresis) into components prior to detecting the emission level. Thus, in contrast to the identification technique disclosed by R.E. Silman in EP-A-0,077,149 (wherein a mix of emissive products is separated and the pattern of the separate signals from emissive components is detected) it is possible in the present invention simply to detect the signal that is the sum of the signals from the various radiolabelled proteins (or other emissive products) in the residue.

It will be understood, of course, that for instance in qualitative determinations, it is possible to use only a portion of the residue in the step of detecting the emission (the remainder of the residue being, for example, subjected to some other procedure, e.g. the above-mentioned identification technique disclosed by R.E. Silman).

It was originally thought that the $^{35}$S methionine or other emissive agent was incorporated into bacterial proteins and that, after precipitation with TCA or the like, the proteins were retained on the glass-fibre paper after filtration. However, in the method described above, using a reagent strip or dipstick, there is no precipitation. Accordingly, it seems likely that the bacterial products are adsorbed on the surface of the glass fibres, that is to say the proteins (containing $^{35}$S methionine) adhere to the fibres despite the washing step, whereas the free $^{35}$S methionine is washed off the paper. An alternative explanation is that the bacteria as a whole adhere to the glass-fibre material, incorporate at least part of the $^{35}$S methionine and subsequently resist being washed away or broken up. Of course, the foregoing explanations are not to be regarded as in any way limiting the present invention since the practice thereof does not depend upon an understanding of the metabolic or other mechanisms involved.

It will of course be understood that the present invention has been described above purely by way of example, and modifications of detail can be made within the scope and spirit of the invention.

0124285

- 28 -

CLAIMS

1. A method of determining the presence of microorganisms in a sample, which method comprises the steps of bringing an emissive agent into contact with the sample; separating off any excess emissive agent; and detecting the level of emission from at least part of the residue.

2. A method according to claim 1, characterised in that the emissive agent is radioactive.

3. A method according to claim 2, characterised in that the emissive agent is selected from $^{35}$S methionine, $^{14}$C leucine and other radioactive amino acids.

4. A method according to claim 1, characterised in that the sample is incubated with the emissive agent prior to the separating step.

5. A method according to claim 1, characterised in that the sample and the emissive agent are brought into contact by forming a mixture containing them.

6. A method according to claim 1, characterised in that the sample is one of a number of samples from the same source, the method comprising the additional step, prior to bringing the sample and emissive agent into contact, of adding to the sample an antibiotic agent selected from a series of such agents, whereby the detected emission levels for the series of samples provides an indication of the drug sensitivity of the microorganism should such be present in the samples.

7. A method according to claim 1, characterised in that the sample is prepared by adding a standard microorganism to a specimen having an unknown level of antibiotic agent, and the detected level of emission is compared with that of a control in order to determine the inhibition of the standard microorganism by the specimen, whereby the level of antibiotic agent in that specimen may be estimated.

8. A device for determining the presence of microorganisms in a sample, characterised in that it comprises an emissive agent and a solid carrier for that emissive agent.

9. A device according to claim 8, characterised in that the said carrier is an absorbent member, which member is impregnated with an emissive agent.

10. A device according to claim 8, characterised in that the emissive agent is selected from $^{35}$S methionine, $^{14}$C leucine and other radioactively labelled amino acids.

11. A device according to claim 10, characterised in that the absorbent member is a non-woven, glass-fibre material.

12. A device according to claim 1, characterised in that the solid carrier is in the form of a strip and/or is associated with a support.

FIG.1.

FIG.2.

0124285

3/3

FIG.3.